# EUROPEAN PATENT SPECIFICATION

(11) **EP 3 090 703 B1**
(45) Date of publication and mention of the grant of the patent: **23.12.2020**
(21) Application number: 14877467.2
(22) Date of filing: 24.03.2014
(51) Int. Cl.: A61F 2/24

(54) **ARTIFICIAL HEART VALVE ANNULOPLASTY RING**
KÜNSTLICHER HERZKLAPPENANNULOPLASTIERING
ANNEAU D'ANNULOPLASTIE DE VALVULE CARDIAQUE ARTIFICIELLE

(30) Priority: 31.12.2013 CN 201310751680
(43) Date of publication of application: 09.11.2016
(73) Proprietor: KINGSTRONBIO(CHANGSHU) CO., LTD, Changshu, Jiangsu 215500 (CN)
(72) Inventor: JIN, Chang, Changshu Jiangsu 215500 (CN); ZHONG, Shengping Sam, Changshu Jiangsu 215500 (CN)
(74) Representative: J A Kemp LLP
(86) International application number: PCT/CN2014/073973
(87) International publication number: WO 2015/100857

(56) References cited:
- EP-A1- 1 348 406
- WO-A1-2009/137776
- CN-A- 102 341 063
- KR-A- 20040 092 842
- US-A1- 2010 211 166
- US-A1- 2012 136 436
- US-A1- 2013 131 792
- US-A1- 2013 226 289
- US-B2- 7 192 442

## Description

### TECHNICAL FIELD

The present disclosure relates to an artificial heart valve annuloplasty ring, belonging to the technical field of medical appliances.

### BACKGROUND

During heart systole, its mitral valve and tricuspid valve close up, forcing intraventricular blood to flow into arteries, and meanwhile preventing it from reversing flow into atriums. However, backflow of flood into the atriums occurs when contraction of ventricles fails to full closure of the valve cusps due to diseases such as valve sinus dilation, rapture of mitral chordae tendinae, perforation of valve cusp or prolapse of valve cusp. Annuloplasty ring implantation, as an important clinical means to reshape diseased mitral valve and tricuspid valve to maintain their normal shape and outline, can be employed to repair ventricular regurgitation due to disease-induced incomplete closure of the mitral valve and tricuspid valve cusps.

Currently, the annuloplasty ring already used for clinical purposes is mainly divided into three types: soft ring, rigid ring and semi-rigid ring. The soft ring is of a two-dimensional structure and a main structure thereof is mainly made of a polymer material, allowing a satisfying fit for an anulus of a valve, but the material of the soft ring is excessively soft, which is not favorable to effective closure of the valve cusps and effective shaping of an anulus. And the rigid ring the semi-rigid ring are of a two-dimensional planar structure or of a three-dimensional saddle structure designed according to anulus of the valve, in which the latter structure has a lot of advantages in reducing stress imposed on the valve cusps and enhancing the durability. The rigid ring usually adopts a metal material and has good support strength, but the rigid ring is not easy to bend, therefore it is unable to achieve a coordinated movement with the cardiac cycle. The semi-rigid ring combines the advantage of both the soft ring and the rigid ring, and can offer good support strength through adjusting rigidity of main body of the annuloplasty ring, and meanwhile effectively adapt to variation of the annulus of the valve caused by cardiac systole and diastole, which bringing the semi-rigid ring into limelight.

At present, regarding the implementation of rigidity adjustment of the semi-rigid ring, the patent NO. US5607471 discloses an annuloplasty ring which takes an variable-diameter wire as its annular main body structure, and adjust rigidity of its various ring sections through adjustment of the wire diameter, however, the design and manufacture of such structure of the mold is tedious, and the manufacturing process thereof is complicated; US6908482 and its patent family members disclose an annuloplasty ring, the main body of which uses a metal strip that is directly made into the outline of the annuloplasty ring, and the requirements for different rigidities is achieved by adjusting the number of the layer of the strip, and the annuloplasty ring prepared with this method presents a large deformation rigidity at the part where a anterior ring joins a posterior ring in a direction perpendicular to the ring plane. Patent US7220277 discloses an annuloplasty ring, its annular main body structure adopts a tube with a circular or other shaped hole hollowed out thereon, the rigidity of the annuloplasty ring is adjusted according to the shape, size and position of the hole, however, the annuloplasty ring is apt to break and crack under a certain force. Alternatively, some annular main bodies adopt a combination of metal and polymer to adjust the rigidity of the entire annuloplasty ring, which requires the metallic ring section and the polymeric ring section to be matched according to rigidity requirement before being connected; and due to special requirements for its application environment, it needs to strictly control the processing of the metallic and polymeric ring sections and connection procedures therebetween, which leads to a complex process and an increased risk of failure , and furthermore, for some of such structure designs, wear and tear often occur at the junction of the metallic and the polymeric ring sections during the course of application, thereby influencing the long-term use effect. Patent WO2009/137776A1 discloses an annuloplasty ring, each ring has a structural ring body with a shape that complies with predicted shapes of degenerative valvular diseases, the layered band structure has varying degrees of stiffness across different oblique planes. Patent US2010/0211166A1 discloses an annuloplasty structure, which has a outer body portion of wire coil.

### SUMMARY

The present disclosure provides an artificial heart valve annuloplasty ring, which can meet the requirements for the application, has easily adjustable rigidity and simple structure, is easy to manufacture, and can maintain a long-term stable plasty effect.

The present disclosure provides an artificial heart valve annuloplasty ring, including an outer layer and an annular main body structure, the outer layer being covered on the periphery of the annular main body structure, wherein, the annular main body structure is at least one layer of a tubular helical structure, and the tubular helical structure is a multi-helical structure formed by curling multiple sheet material strips, and the tubular helical structure can adjust rigidity of the annuloplasty ring.

The annuloplasty ring may have a main body with a shape similar to that in the prior art, i.e., the main body may be integrally formed by multiple ring sections, including an anterior ring section, a posterior ring section, a left ring section, a right ring section and connecting sections arranged therebetween, and the ring sections form an integrated structure and together form a closed or open annular main body. The annular main body is externally covered with a biocompatible fiber fabric layer, and the fiber fabric layer may be a polymer material meeting medical standards, for instance, a medical polyester fabric. The tubular helical structure is a structure formed by curling multiple sheet material strips axially and generally is tubular.

According to the artificial heart valve annuloplasty ring provided by the present disclosure, the tubular helical structure is a multi-helical structure formed by curling multiple sheet material strips, where the multi-helical structure may comprise multiple single-helical structures with the same or different helical directions.

Not all helical structures in the multi-helical structure have the same helical pitch.

According to the artificial heart valve annuloplasty ring provided by the present disclosure, in the case of the tubular helical structure being a multi-helical structure formed by curling multiple sheet material strips, not all the multiple sheet material strips have the same material, or not all the multiple sheet material strips have the same width, which is conducive to further change and adjust the rigidity of the annular main body.

According to the artificial heart valve annuloplasty ring provided by the present disclosure, not all portions of the sheet material strips have the same width, and portions with different widths may be connected in stepwise form or gradual transition form.

According to the artificial heart valve annuloplasty ring provided by the present disclosure, the tubular helical structure is a multi-helical structure formed by curling multiple sheet material strips, every two of which are connected via at least one connecting portion. The connection may be implemented through direct welding or welding by reinforcing rib, so as to facilitate increasing the local strength as well as coordination of multiple sheet material strips during movement.

According to the artificial heart valve annuloplasty ring provided by the present disclosure, the sheet material strips may have slotted structures, so as to further change and adjust the rigidity of the annular main body. There are no particular requirements for the specific arrangement of the slotted structures, which may be through holes with different shapes, for example, round, oval, or polygon, such as triangle, square, and other shape, or a combination thereof. Of course, taking into account of processing convenience and assurance of characteristics of the tubular helical structure, it may choice through holes having a definite shape or through holes varied little in shape. According to the artificial heart valve annuloplasty ring provided by the present disclosure, for a single sheet material strip, not all portions thereof have the same slotted structure; or for different sheet strips, not all of them have the same slotted structure.

According to the artificial heart valve annuloplasty ring provided by the present disclosure, the tubular helical structure is formed by at least two layers, with one sleeved over another, and each layer of the tubular helical structure is formed by curling the sheet material strips. In the present disclosure, the tubular helical structure of the annular main body may be a single-layered structure, or alternatively a double-layered or multi-layered structure by inserting a tubular helical structure with a smaller external diameter into a tubular helical structure with a larger internal diameter. For an annular main body with a double-layered or a multi-layered tubular helical structure, the same portion of each of the layers may adopt the same or different hollow-out rates, pitches or widths of the sheet material strips, so as to satisfy different demands for the rigidity of different ring sections, and thereby to allow the entire annuloplasty ring to be perfectly matched with the cardiac cycle.

According to the artificial heart valve annuloplasty ring provided by the present disclosure, the tubular helical structure is internally provided with a wire capable of assisting in adjusting the rigidity of the annuloplasty ring. In particular, in the case of an annular main body structure with a high slotted rate, in order to keep the length and shape of the annular main body structure relatively fixed, the annular main body structure may be arranged with a wire for assisting in adjusting the rigidity of the annuloplasty ring. The wire usually has a length that is not longer than that of the tubular helical structure, and thereby may be arranged in the tubular helical structure, and the wire may be continuous, or arranged in the tubular helical structure in a spaced manner. The use of the wire can effectively prevent changes in the structure of the tubular helical structure during three-dimensional shaping and application, and limit extension and contraction lengths of the annuloplasty ring, so as to avoid the case that the annuloplasty ring cannot effectively limit the valve cusps after prolonged use. The wire may be a medical metal material or medical polymer material and may be arranged in a conventional method, and the present disclosure makes no limitation on the method.

According to the artificial heart valve annuloplasty ring provided by the present disclosure, a heat shrinkable tube is arranged between the outer layer and the tubular helical structure. The arrangement of the heat shrinkable tube further contributes to three-dimensional shaping of the tubular helical structure and preventing changes in the structure thereof during the use. The heat shrinkable tube may generally be a polymer material, and is sleeved over the periphery of the tubular helical structure via a heat shrinking process.

According to the artificial heart valve annuloplasty ring provided by the present disclosure, gaps between the sheet material strips of the tubular helical structure are filled with a polymer material. For instance, polymer material different from the material of the sheet material strips may be filled in the gaps between the sheet material strips of the tubular helical structure, through an injection molding method, or soaking the tubular helical structure in a solution or a colloidial solution of the polymer and then drying, to form a tubular helical structure with different materials spaced apart.

According to the artificial heart valve annuloplasty ring provided by the present disclosure, the outer diameter of the tubular helical structure may be 0.6-3 mm. With respect to the annuloplasty ring with multiple layers of the tubular helical structure, the outer diameter may be interpreted as the outer diameter of the outmost layer of the tubular helical structure.

According to the artificial heart valve annuloplasty ring provided by the present disclosure, the sheet material strips may have a thickness of 0.1-1 mm. With respect to the annuloplasty ring with multiple layers of the tubular helical structure, the thickness may be interpreted as the sum of thicknesses of the sheet material strips of the multiple layers of the tubular helical structure.

In the present disclosure, the width, slotted rate and thickness of the sheet material strips of the multiple ring sections of the annular main body, as well as the pitch, the number of the layer and the outer diameter of the tubular helical structure may be adjusted individually or synergistically to obtain the annuloplasty ring with different rigidities. For example, at the time when an overall rigidity of the annuloplasty ring is obtained through selecting and determining proper width and thickness of the sheet material strips, as well as proper pitch, the number of the layer and tube diameter of the tubular helical structure, the sheet material strips for forming the tubular helical structure are hollowed out, and through adjusting the slotted rate (i.e. adjusting pattern, location and/or density of the slotted structure), the rigidity of the annular main body can be further adjusted. The slotted structure may be arranged on each ring section, or selectively arranged on one or several ring sections; and the slotted rate of the slotted structures on different ring sections may be the same or different, and it is understood that various ring sections of the annuloplasty ring having a rigidity that is not the same or not all the same is more beneficial to clinical applicaiton. Alternatively, requirements for different rigidities of various ring sections of the annuloplasty ring may be met by combing with adjustment of the pitch. For instance, for a ring section requiring a higher rigidity, the corresponding sheet material strip adopts a low slotted rate or does not perform a hollowing process, and also adopts a small pitch; for a ring section requiring a lower rigidity, the corresponding sheet material strip adopts a high slotted rate and a big pitch.

The "slotted rate" used by the present disclosure refers to the percentage of the slotted area formed on the sheet material strip accounting for the area of the sheet material strip, and may be realized through designs of the shape, size and distribution of openings.

In the present disclosure, forming the slotted structure on the sheet material strip may be conducted via any feasible methods, for instance, a preferred method for a metal sheet material strip is laser cutting. The annuloplasty ring in the present disclosure may be manufactured by first cutting a sheet material strip having a desired length, then cutting a pattern with desired density and structure on the sheet material strip via laser cutting or other methods, and then shaping with a mold to form a final structure; or, after shaping to form the final structure, processing the final structure through a three-dimensional soft laser cutting or other methods to obtain a slotted pattern with the desired density and structure. The above method eliminates the need for re-designing and processing a mold for the main body of the annuloplasty ring when adjusting the rigidity of the annuloplasty ring, and the method is simple and convenient.

According to the artificial heart valve annuloplasty ring provided by the present disclosure, the sheet material strip may use various medical alloys or polymers, including medical nickel titanium alloy, cobalt chromium alloy, titanium alloy, stainless steel or polymers. For example, the sheet material strip may be made of stainless steel 316L, cobalt chromium alloy MP35, L605, Elgiloy® or Phynox®, titanium alloy Ti6A14V, nickel titanium alloy Ni-Ti, or polymers, such as polyethylene, polytetrafluoroethylene, polyformaldehyde resin or polyethylene terephthalate.

According to the artificial heart valve annuloplasty ring provided by the present disclosure, an intermediate layer may be arranged between the outer layer and the annular main body. The intermediate layer is usually made of silicone, which facilitates suture the annuloplasty ring and the heart together, and meanwhile allowing the inner tube structure to be easier to bend and rebound, thus fixing and effectively stabilizing the tube structure. The intermediate layer may adopt a circular tube that is directly sleeved over the annular main body, or a coating, heat shrinkable tube or heat shrinkable film that are fixed on the outside of the annular main body.

Implementation of the solutions of the present disclosure at least has the following advantages:

1. The artificial heart valve annuloplasty ring provided by the present disclosure adopts a tubular helical structure as an annular main body formed by curling a sheet material strip, is capable of providing various ring sections with different rigidities, has a simple structure, readily adjustable rigidity, and thus can better adapt to movement characteristics of a mitral valve or a tricuspid valve;

2. The artificial heart valve-repairing annuloplasty ring of the present disclosure eliminates the need for redesigning and processing a mold for the main body of the annuloplasty ring when adjusting rigidity of the annuloplasty ring, and has better durability, and the processing method thereof is easy, convenient and has high productivity.

### BRIEF DESCRIPTION OF THE DRAWINGS

FIG. 1 is a diagram of a mitral valve annuloplasty ring provided by an embodiment of the present disclosure.
FIG. 2 is an unfolded diagram of a tubular helical structure of the main body of a mitral valve annuloplasty ring provided by an embodiment of the present disclosure.
FIG. 3 is a schematic structure diagram of the cross section of a mitral valve annuloplasty ring provided by an embodiment of the present disclosure.
FIG. 4 is a diagram of a sheet material strip for forming the tubular helical structure shown in FIG. 2.
FIG. 5 is a diagram of a tubular helical structure of the main body of a mitral valve annuloplasty ring provided by another embodiment of the present disclosure.
FIG. 6 is a diagram of a sheet material strip for forming the tubular helical structure shown in FIG. 5.
FIG. 7 is a diagram of a tubular helical structure of the main body of a mitral valve annuloplasty ring provided by another embodiment of the present disclosure.
FIG. 8 is a schematic structure diagram of the cross section of a mitral valve annuloplasty ring provided by yet another embodiment of the present disclosure.
FIG. 9 is a diagram of the longitudinal cross section of a close-ring junction portion of a mitral valve annuloplasty ring provided by yet another embodiment of the present disclosure.
FIG. 10 is a diagram of a sheet material strip for forming the tubular helical structure shown in FIG. 7.
FIGS. 11A and 11B are drawings of partial enlargement for the tubular helical structure shown in FIG. 7.
FIG. 12 is a diagram of a tricuspid valve annuloplasty ring provided by another embodiment of the present disclosure.
FIG. 13 is a diagram of a tubular helical structure of the main body of a tricuspid valve annuloplasty ring provided by another embodiment of the present disclosure.
FIG. 14 is a diagram of a sheet material strip for forming the tubular helical structure shown in FIG. 13.

Description for reference numbers in the drawings:
1- anterior ring section; 2- posterior ring section; 3- left ring section; 4- right ring section; 5-connecting section; 6- outer layer; 7- intermediate layer; 8- annular main body structure; 9-wire; 10- lateral ring section

### DESCRIPTION OF EMBODIMENTS

Hereinafter, the present disclosure will be described in further detail combining with specific embodiments and examples, the purpose is to better understand the substantive content of the present disclosure, and cannot be construed as limiting scope of the present invention in any way. The invention is defined by the claims.

The present disclosure provides an annuloplasty ring with different rigidities for different portions. The annuloplasty ring is highly fit for the movement of a mitral valve or a tricuspid valve in directions both perpendicular and parallel to the ring plane, has good durability; and equipment and process for manufacturing the annuloplasty ring are simple, moreover, the rigidity of the annuloplasty ring can be adjusted conveniently.

The contour of the inventive annuloplasty ring may not be different from the artificial heart valve annuloplasty ring currently put into clinical use or recorded, for instance, it may have the same contour with the annuloplasty ring disclosed in patent No. CN101374478A entitled "a valve annuloplasty ring for mitral valve prolapse". The annular main body structure may be processed into an open or closed ring structure with a planar or three-dimensional structure as needed. When the annuloplasty ring is a closed structure, the closed structure may be realized through such as stitching, binding, welding or sleeving of close-ring junction portion of the annuloplasty ring.

The annuloplasty ring usually includes an outer layer and a main body structure. The outer layer commonly adopts a fabric material woven from polyester fiber or polytetrafluoroethylene, which is covered around the periphery of the main body structure; the annular main body structure forms a tubular helical structure by curling a sheet material strip; and the curled tubular helical structure is formed into a desired planar or three-dimensional open or closed ring structure. The annuloplasty ring may further include an intermediate layer that is covered outside the layer of the main body structure; the intermediate layer is often made of silicone, so as to facilitate suture to the heart; the annuloplasty ring may also be not provided with the intermediate layer, and be directly sutured to the heart through the outer layer.

The rigidity of the annuloplasty ring formed by the curled tubular helical structure may be adjusted through adjusting the width of different portions of the sheet material strips used for curling, and slotted patterns and density of the sheet material strips, thickness of the sheet material strips, the pitch after curling, the diameter of the tubular helical structure. For instance, rigidity of the annuloplasty ring may be adjusted by the following methods: in the case of an equal pitch after curling, adjusting the width of the sheet material strips so that a rigid ring section of the annuloplasty ring has a larger width, and a soft ring section of the annuloplasty ring has a smaller width, then shaping the curled tubular helical structure into a desired contour of the annuloplasty ring, to obtain an annuloplasty ring with a desired rigidity; or adjusting the slotted pattern and density of the sheet material strips so that less material is removed from a high-rigidity ring section of the annuloplasty ring, and more material is removed from a soft ring section; or adjusting the thickness of the utilized sheet material strips and the diameter of the curled tubular helical structure; or directly adjusting the pitch so that a soft ring section of the annuloplasty ring has a bigger pitch, and a rigid ring section has a smaller pitch; or adjusting the thickness of the sheet material strips and the diameter of the curled tubular helical structure so that different ring sections of the annuloplasty ring have different rigidities; or using a combination of the above adjusting factors.

The annular main body structure may adopt multiple sheet material strips having the same or different materials, and the sheet material strips may be curled simultaneously and formed a tube with a double-helical or multi-helical structure. The curled sheet material strips may have the same or different helical directions. The double-helical or multiple-helical structure may be welded directly or by reinforcing rib at some points therebetween, so as to increase the local strength and movement coordination between the double-helical structure or the multiple-helical structure. When using multiple sheet material strips to form a double-helical or multi-helical structure, the used sheet material strips may be of the same or different materials, and may have the same or different strip widths, patterns, and wall thicknesses. The material adopted by the main body structure is usually medical stainless steel, cobalt-base alloy, nickel titanium alloy and other metal materials; or polytetrafluoroethylene, polypropylene and other polymer materials.

According to needs, the sheet material strips may be formed into two tubular helical structures with a bigger diameter and a smaller diameter, respectively, then the tubular helical structure with the smaller diameter is placed into the tubular helical structure with the bigger diameter, and finally, they are formed into a planar or three-dimensional open or closed ring structure.

Additionally, according to rigidity and other requirements, a wire may be added into the curled tubular helical structure, and the wire may use a material that is the same as or different from the material of the sheet material strips. For example, a silicone wire is provided in a tubular helical structure made of stainless steel.

Hereinafter, specific embodiments of the present disclosure will be described in conjuction with the drawings.

### Embodiment 1

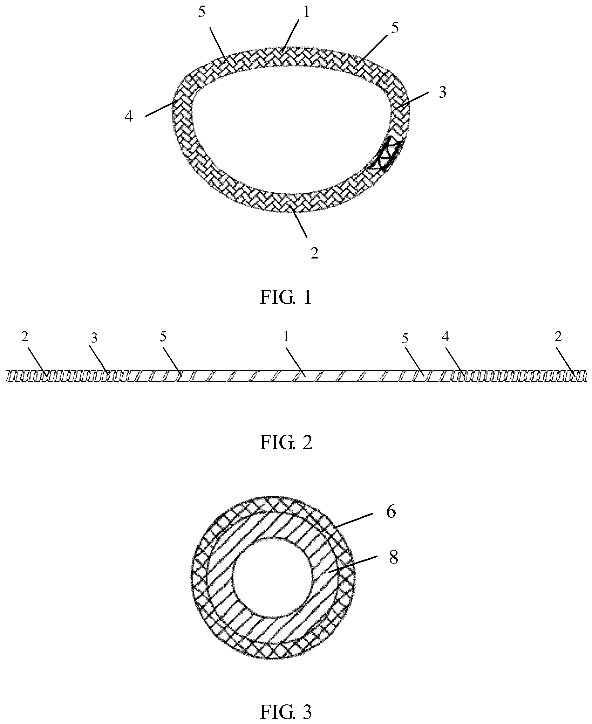

This embodiment provides an artificial mitral valve annuloplasty ring. As shown in FIG. 1, the mitral valve annuloplasty ring is of a closed three dimensional saddle structure, and the annular main body thereof comprises an anterior ring section 1, a posterior ring section 2, a left ring section 3 and a right ring section 4. The higher arched bulge of the annuloplasty ring is the anterior ring section 1, the lower bulge is the posterior ring section 2, two depressed sections are the left ring section 3 and the right ring section 4, respectively; adjacent ring sections among the anterior ring section 1, the left ring section 3 and the right ring section 4 are respectively provided with a connecting section 5.

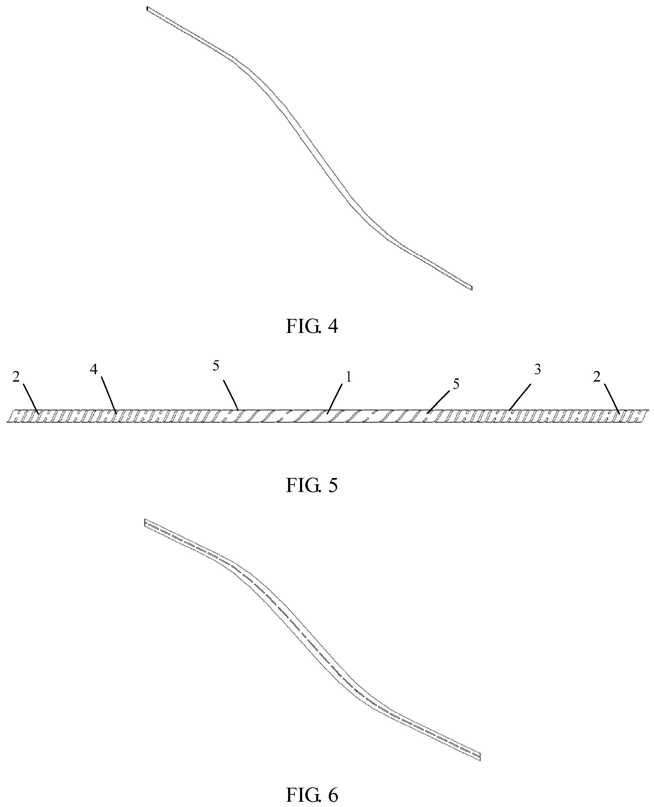

As shown in FIG. 2, taking the middle point of the posterior ring section 2 as a connecting point, the annuloplasty ring is spread along the circumferential direction thereof to form a straight and flat tubular helical structure, and the tubular helical structure may have an outer diameter of 1-3 mm and consistent pitch. A sheet material strip for forming the helical structure has different widths at different portions, and the adopted sheet material strip usually may have a thickness of 0.1-0.6 mm, and is formed through such as laser cutting or stamping. As illustrated in FIG. 4, the sheet material strip has a bigger width at the middle part and a smaller width at two ends. The change between the two different widths is gradually transitional.

Through adjusting the width of the sheet material strip, various ring sections of the annuloplasty ring are adjusted to be beneficial to coordinate movement of the annuloplasty ring with the systolic and diastolic cardiac cycle. As illustrated in FIG. 2, the posterior ring section 2, the left ring section 3 and the right ring section 4 are flexible ring sections, and have the smallest width of the sheet material strip; the anterior ring section 1, compared with the posterior ring section 2, the left ring section 3 and the right ring section 4, is a rigid ring section, and has the largest width of the sheet material strip; and the width of the sheet material strip of the connecting section 5 lies between the widths of the sheet material strips of the two ring sections connected by the connecting section 5.

FIG. 3 is a cross-section diagram of the annuloplasty ring, which uses a tubular helical structure formed by curling a sheet material strip made of medical cobalt chromium alloy, as a main body 8. An outer layer 6 is a medical polyester fiber fabric with good biocompatibility, and can be connected with the heart by suturing during surgery.

The closure connecting end of the tubular helical structure that is made of a medical cobalt chromium alloy and serves as the main body 8, may be connected through welding, thereby realizing closing of the annuloplasty ring; the medical polyester fiber fabric of the outer layer 6 is directly coated outside the tubular helical structure 8 made of medical cobalt chromium alloy via suturing.

In addition, a heat shrinkable tube (not shown) may be arranged between the outer layer 6 and the tubular helical structure. The arrangement of the heat shrinkable tube further facilitates three-dimensional shaping of the tubular helical structure and preventing changes in structure of the tubular helical structure during use. The heat shrinkable tube may usually be made of polymer material, for example, polytetrafluoroethylene, and is sleeved over the outer circumference of the tubular helical structure via a heat shrinking process.

For the artificial mitral valve annuloplasty ring provided by this embodiment, it is realized that the anterior, posterior, left and right portions of the artificial mitral valve annuloplasty ring form four movement units due to different rigidities. The anterior ring section 1 with a bigger width of the sheet material strip (larger rigidity) are mainly used to maintain curvature of the artificial mitral valve annuloplasty ring for simulating physiological mitral valve ring saddle structure, while the posterior ring section 2, the left ring section 3 and the right ring section 4 with a smaller width of the sheet material strip (smaller rigidity) cooperate with the anterior ring section 1 to make the movement mode of the artificial mitral valve annuloplasty ring approach the manner and requirements of the movement of the physiological mitral valve ring, and the connecting section 5 plays a role of transition among the above sections. As a result, the artificial mitral valve annuloplasty ring of this embodiment can achieve a coordinated movement with each cardiac cycle as the heart throbs.

### Embodiment 2

This embodiment provides an artificial mitral valve annuloplasty ring. FIG. 5 is a diagram of spreading an annular main body of the annuloplasty ring along the circumference of the ring into a straight flat tubular helical structure. FIG. 6 is a sheet material strip for forming the tubular helical structure shown in FIG.5. The sheet material strip in this embodiment is produced by processing a medical stainless steel plate, has a width larger than the sheet material strip in embodiment 1, and is provided with multiple long strip-shaped slotted structures. Other than that, the other shapes and structures of the artificial mitral valve annuloplasty ring provided by this embodiment are the same as those of the artificial mitral valve annuloplasty ring in embodiment 1. Through arranging the hollow-out structure, the present disclosure realizes further adjustment of rigidities of various ring sections.

### Embodiment 3

This embodiment provides an artificial mitral valve annuloplasty ring, as shown in FIG. 1, having the same shape as the artificial mitral valve annuloplasty ring in embodiment 1.

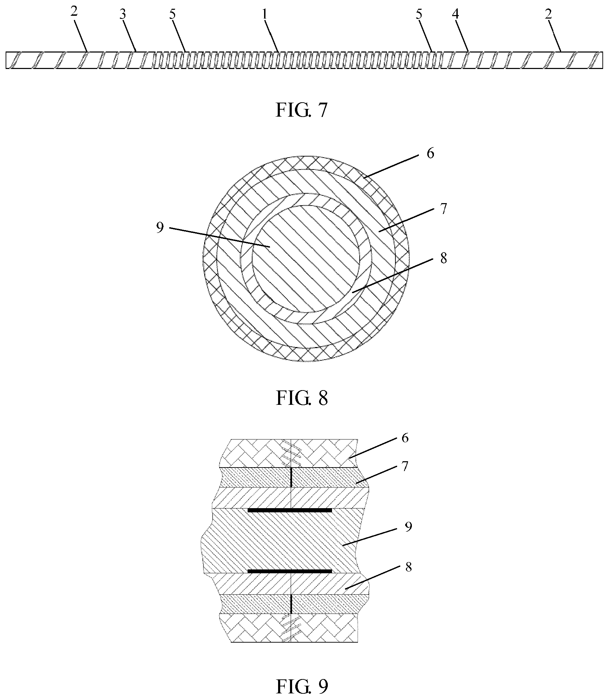

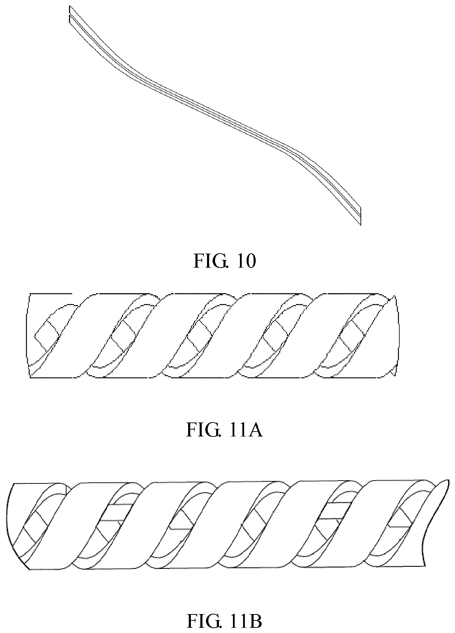

As shown in FIG. 7, taking the middle point of the posterior ring section 2 as a closure connecting point, the main body 8 of the annuloplasty ring is spread along a circumference of the ring into a straight flat tubular helical structure, the tubular helical structure may have an outer diameter of 1-3 mm and consistent pitch. A sheet material strip for forming the helical structure has different widths at different portions, and usually a thickness of 0.1-0.6 mm, and the sheet material strip is formed by for example laser cutting or stamping. FIG. 10 shows a sheet material strip for forming the main body structure by curling, and the main body structure adopts a double-sheet material strip, as shown in the figure, for curling into a double-helical structure, which can increase anti-fatigue capacity of the sheet material strip. As shown in FIG. 10, the sheet material strip has a smaller width at the middle part and a larger width at two ends. The change between the two different widths is gradually transitional. FIG. 11A is a local diagram of the curled double-helical structure, and connection bars may be welded at certain parts of the two helical structures with reference to FIG. 11B, so as to enhance movement coordination between different helical structures.

The anterior ring section 1, the left ring section 3 and the right ring section 4 are flexible, with the smallest width of the sheet material strip; the posterior ring section 4, compared with the anterior ring section 1, the left ring section 3 and the right ring section 4, is rigid, with the largest width of the sheet material strip; and width of the sheet material strip of the connecting section 5 lies between the widths of the sheet material strips of two ring sections connected by the connecting section 5.

As shown in FIG. 8, the artificial mitral valve annuloplasty ring provided by this embodiment has an outmost layer 6 made of a polyester fiber fabric, and has an intermediate layer 7 made of silicone tube, the main body 8 is formed by curling a sheet material strip made of a medical cobalt chromium alloy, and the tubular helical structure is internally provided with a cobalt chromium alloy wire 9.

FIG. 9 is a longitudinal cross-section diagram of a closure connecting part of the annuloplasty ring. The tubular helical structure that is made of a medical cobalt chromium alloy and serves as the main body 8, is directly welded to the wire 9 made of cobalt chromium alloy; the silicone tube as the intermediate layer 7 is tightly sleeved over the outside of the tubular helical structure 8 made of a medical nickel titanium alloy. Joints are butted against each other and bonded by using a biocompatible adhesive. And the outer layer 6 is a medical polyester fiber fabric, and is directly stitched to coat the outside of the silicone tube 7.

The mitral valve annuloplasty ring provided by this embodiment may help to maintain a physiological saddle shape which changes with the cardiac cycle, and ensure involution quality of the valve cusps, and meanwhile, the use of the wire can effectively prevent changes in the structure during three-dimensional shaping and application after the annuloplasty ring has been cut, and limit the maximum extension and contraction lengths of the annuloplasty ring, avoiding that the valve cusps cannot be effectively limited after long-term use.

### Embodiment 4

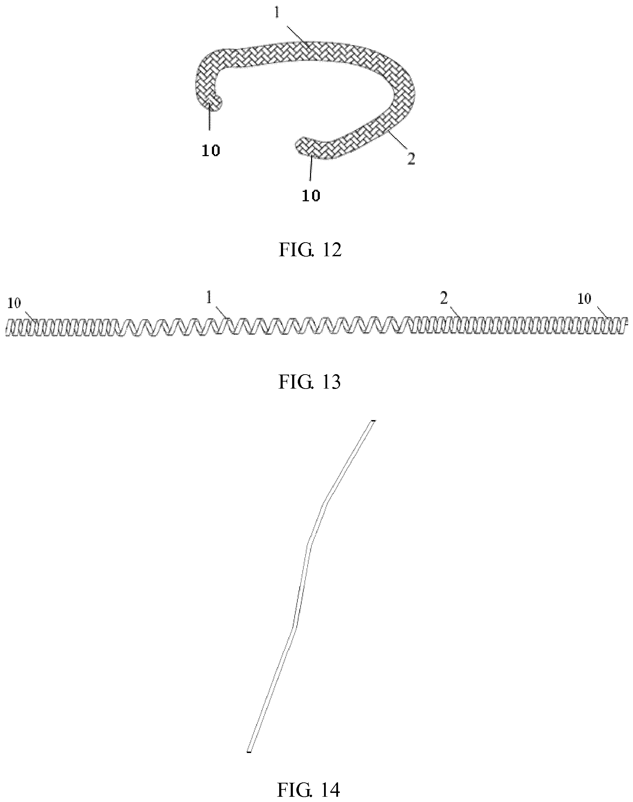

This embodiment provides an artificial tricuspid valve annuloplasty ring, which has a three-dimensional structure fit for physiological shaping of a tricuspid valve, as shown in FIG. 12, and comprises an anterior ring section 1, a posterior ring section 2 and a lateral ring section 10.

As shown in FIG. 13, the annuloplasty ring is spread along the circumference of the ring into a straight flat tubular helical structure. As shown in FIG. 14, a sheet material strip for curling into the tubular helical structure has consistent width at various portions, and the rigidity of the tubular helical structure may be further adjusted through adjusting pitches between different ring sections of the tubular helical structure. Where, the anterior ring section 1 has a large pitch and a low rigidity, and the posterior ring section 2 and part of the lateral ring section 10 have a small pitch and a high rigidity.

In the artificial tricuspid valve annuloplasty ring provided by this embodiment, the outer layer 6 is a polyester fiber fabric, and the intermediate layer 7 is a silicone tube with an inner diameter greater than the outer diameter of the tubular helical structure of the main body. The main body 8 is a tubular helical structure made of a medical nickel titanium alloy. Two ends of the intermediate layer 7 and the outer layer 6 are directly stitched for wrapping the tubular helical structure 8 as the main body therein.

Additionally, gaps between sheet material strips of the tubular helical structure may be filled with a polymer material, such as silicone. This may be done as follows: filling a cylindrical material within the tubular helical structure in a removable manner, soaking the tubular helical structure into a silicone solution, drying, and then drawing the internally filled cylindrical material out, to form a tubular helical structure where the silicone and the medical nickel titanium alloy are spaced apart from each other. Alternatively, the tubular helical structure may be directly soaked into the silicone solution without filling with the cylindrical material, and then drying, to form a tubular helical structure where the internal thereof is filled with the silicone and the outside thereof is the silicone and the medical nickel titanium alloy which are spaced apart from each other.

The tricuspid valve annuloplasty ring provided by this embodiment may help to maintain a physiological three-dimensional shape which changes with the cardiac cycle, and ensure involution quality of the valve cusps, and thus is capable of being employed in clinical shaping repair surgeries of a tricuspid valve, so that the tricuspid valve is able to achieve a coordinated movement with the cardiac cycle.

Finally, it should be noted that the above embodiments are merely provided for describing rather than limiting the technical solutions of the present invention. It should be understood by persons skilled in the art that modifications can be made to the technical solutions described in the foregoing embodiments, or equivalent replacements can be made to part or all technical features in the technical solutions; provided that such modifications or replacements do not cause the corresponding technical solutions to depart from the scope of the present invention as defined by the claims.

## Claims

1. An artificial heart valve annuloplasty ring, comprising an outer layer (6) and an annular main body structure (8), the outer layer (6) being covered on the periphery of the annular main body structure (8), **characterized in that**, the annular main body structure (8) is at least one layer of a tubular helical structure, and the tubular helical structure is a multi-helical structure formed by curling multiple sheet material strips, and the tubular helical structure can adjust rigidity of the annuloplasty ring.

2. The artificial heart valve annuloplasty ring according to claim 1, wherein not all helical structures in the multi-helical structure have the same helical pitch.

3. The artificial heart valve annuloplasty ring according to claim 1 or 2, wherein not all the multiple sheet material strips are of the same material.

4. The artificial heart valve annuloplasty ring according to claim 1 or 2, wherein not all the multiple sheet material strips have the same width.

5. The artificial heart valve annuloplasty ring according to claim 1 or 2, wherein the tubular helical structure is formed by at least two layers, with one sleeved over another, and each layer of the tubular helical structure is formed by curling the sheet material strips.

6. The artificial heart valve annuloplasty ring according to claim 1 or 2, wherein various portions of the sheet material strips have different widths, and portions with different widths are connected in stepwise form or gradual transition form.

7. The artificial heart valve annuloplasty ring according to claim 1 or 2, wherein every two of the sheet material strips are connected via at least one connecting portion.

8. The artificial heart valve annuloplasty ring according to claim 1 or 2, wherein the sheet material strips are provided with slotted structures.

9. The artificial heart valve annuloplasty ring according to claim 8, wherein for a single sheet material strip, not all portions thereof have the same slotted structure; or for different sheet material strips, not all of them have the same slooted structure.

10. The artificial heart valve annuloplasty ring according to claim 1, wherein the tubular helical structure is internally provided with a wire (9) capable of assisting in adjusting the rigidity of the annuloplasty ring.

11. The artificial heart valve annuloplasty ring according to claim 1, wherein a heat shrinkable tube is further arranged between the outer layer and the tubular helical structure.

12. The artificial heart valve annuloplasty ring according to claim 1, wherein gaps between the sheet material strips of the tubular helical structure are filled with a polymer material.

13. The artificial heart valve annuloplasty ring according to claim 1, wherein the tubular helical structure has an outer diameter of 0.6-3 mm.

14. The artificial heart valve annuloplasty ring according to claim 1, wherein the sheet material strips have a thickness of 0.1-1 mm.

## Patentansprüche

1. Künstlicher Herzklappen-Annuloplastiering, umfassend eine Außenschicht (6) und eine ringförmige Hauptkörperstruktur (8), wobei die Außenschicht (6) am Umfang der ringförmigen Hauptkörperstruktur (8) bedeckt ist, **dadurch gekennzeichnet, dass** die ringförmige Hauptkörperstruktur (8) mindestens eine Schicht einer rohrförmigen Helixstruktur ist und die rohrförmige Helixstruktur eine mehrfache Helixstruktur ist, gebildet durch Winden von mehreren Plattenmaterialstreifen, und die rohrförmige Helixstruktur Steifigkeit des Annuloplastierings anpassen kann.

2. Künstlicher Herzklappen-Annuloplastiering nach Anspruch 1, wobei nicht alle Helixstrukturen in der mehrfachen Helixstruktur die gleiche Helixganghöhe haben.

3. Künstlicher Herzklappen-Annuloplastiering nach Anspruch 1 oder 2, wobei nicht alle mehrere Plattenmaterialstreifen aus dem gleichen Material sind.

4. Künstlicher Herzklappen-Annuloplastiering nach Anspruch 1 oder 2, wobei nicht alle mehrere Plattenmaterialstreifen die gleiche Breite haben.

5. Künstlicher Herzklappen-Annuloplastiering nach Anspruch 1 oder 2, wobei die rohrförmige Helixstruktur durch mindestens zwei Schichten gebildet ist, mit einer über eine andere geschoben, und jede Schicht der rohrförmigen Helixstruktur durch Winden der Plattenmaterialstreifen gebildet ist.

6. Künstlicher Herzklappen-Annuloplastiering nach Anspruch 1 oder 2, wobei verschiedene Abschnitte der Plattenmaterialstreifen unterschiedliche Breiten haben und Abschnitte mit unterschiedlichen Breiten in schrittweiser Form oder gradueller Übergangsform verbunden sind.

7. Künstlicher Herzklappen-Annuloplastiering nach Anspruch 1 oder 2, wobei jeder zweite der Plattenmaterialstreifen über mindestens einen Verbindungsabschnitt verbunden ist.

8. Künstlicher Herzklappen-Annuloplastiering nach Anspruch 1 oder 2, wobei die Plattenmaterialstreifen mit geschlitzten Strukturen bereitgestellt sind.

9. Künstlicher Herzklappen-Annuloplastiering nach Anspruch 8, wobei für einen einzelnen Plattenmaterialstreifen nicht alle Abschnitte davon die gleiche geschlitzte Struktur haben; oder für unterschiedliche Plattenmaterialstreifen nicht alle davon die gleiche geschlitzte Struktur haben.

10. Künstlicher Herzklappen-Annuloplastiering nach Anspruch 1, wobei die rohrförmige Helixstruktur intern mit einem Draht (9) bereitgestellt ist, der in der Lage ist, beim Anpassen der Steifigkeit des Annuloplastierings zu helfen.

11. Künstlicher Herzklappen-Annuloplastiering nach Anspruch 1, wobei ein Schrumpfschlauch ferner zwischen der Außenschicht und der rohrförmigen Helixstruktur angeordnet ist.

12. Künstlicher Herzklappen-Annuloplastiering nach Anspruch 1, wobei Lücken zwischen den Plattenmaterialstreifen der rohrförmigen Helixstruktur mit einem Polymermaterial gefüllt sind.

13. Künstlicher Herzklappen-Annuloplastiering nach Anspruch 1, wobei die rohrförmige Helixstruktur einen Außendurchmesser von 0,6-3 mm hat.

14. Künstlicher Herzklappen-Annuloplastiering nach Anspruch 1, wobei die Plattenmaterialstreifen eine Dicke von 0,1-1 mm haben.

## Revendications

1. Anneau d'annuloplastie de valve cardiaque artificielle, comprenant une couche extérieure (6) et une structure de corporelle principale annulaire (8), la couche extérieure (6) étant couverte sur la périphérie de la structure de corporelle principale annulaire (8), **caractérisé en ce que** la structure de corporelle principale annulaire (8) est au moins une couche d'une structure hélicoïdale tubulaire, et la structure hélicoïdale tubulaire est une structure multi-hélicoïdale formée en bouclant de multiples bandes de matériau en feuille, et la structure hélicoïdale tubulaire peut ajuster la rigidité de l'anneau d'annuloplastie.

2. Anneau d'annuloplastie de valve cardiaque artificielle selon la revendication 1, dans lequel toutes les structures hélicoïdales dans la structure multi-hélicoïdale n'ont pas le même pas hélicoïdal.

3. Anneau d'annuloplastie de valve cardiaque artificielle selon la revendication 1 ou 2, dans lequel toutes les multiples bandes de matériau en feuille ne sont pas du même matériau.

4. Anneau d'annuloplastie de valve cardiaque artificielle selon la revendication 1 ou 2, dans lequel toutes les multiples bandes de matériau en feuille n'ont pas la même largeur.

5. Anneau d'annuloplastie de valve cardiaque artificielle selon la revendication 1 ou 2, dans lequel la structure hélicoïdale tubulaire est formée par au moins deux couches, une étant emmanchée sur une autre, et chaque couche de la structure hélicoïdale tubulaire est formée en bouclant les bandes de matériau en feuille.

6. Anneau d'annuloplastie de valve cardiaque artificielle selon la revendication 1 ou 2, dans lequel diverses parties des bandes de matériau en feuille ont des largeurs différentes, et des parties avec des largeurs différentes sont raccordées sous forme progressive ou forme de transition graduelle.

7. Anneau d'annuloplastie de valve cardiaque artificielle selon la revendication 1 ou 2, dans lequel chaque paire des bandes de matériau en feuille est raccordée par l'intermédiaire d'au moins une partie de raccordement.

8. Anneau d'annuloplastie de valve cardiaque artificielle selon la revendication 1 ou 2, dans lequel les bandes de matériau en feuille sont pourvues de structures rainurée.

9. Anneau d'annuloplastie de valve cardiaque artificielle selon la revendication 8, dans lequel, pour une seule bande de matériau en feuille, toutes les parties de celle-ci n'ont pas la même structure rainurée ; ou pour différentes bandes de matériau en feuille, toutes n'ont pas la même structure rainurée.

10. Anneau d'annuloplastie de valve cardiaque artificielle selon la revendication 1, dans lequel la structure hélicoïdale tubulaire est pourvue, de façon interne, d'un fil (9) capable d'aider l'ajustement de la rigidité de l'anneau d'annuloplastie.

11. Anneau d'annuloplastie de valve cardiaque artificielle selon la revendication 1, dans lequel un tube thermo-rétrécissable est en outre agencé entre la couche extérieure et la structure hélicoïdale tubulaire.

12. Anneau d'annuloplastie de valve cardiaque artificielle selon la revendication 1, dans lequel des espaces entre les bandes de matériau en feuille de la structure hélicoïdale tubulaire sont remplis avec un matériau polymère.

13. Anneau d'annuloplastie de valve cardiaque artificielle selon la revendication 1, dans lequel la structure hélicoïdale tubulaire a un diamètre extérieur de 0,6 à 3 mm.

14. Anneau d'annuloplastie de valve cardiaque artificielle selon la revendication 1, dans lequel les bandes de matériau en feuille ont une épaisseur de 0,1 à 1 mm.
